# EUROPEAN PATENT APPLICATION

(11) **EP 3 112 922 A1**
(43) Date of publication of application: **04.01.2017**
(21) Application number: 15306047.0
(22) Date of filing: 30.06.2015
(51) Int. Cl.: G02B 27/01, A61B 3/113, G06F 3/01, G02B 27/00

(54) **A GAZE TRACKING DEVICE AND A HEAD MOUNTED DEVICE EMBEDDING SAID GAZE TRACKING DEVICE**

(71) Applicant: Thomson Licensing, 92130 Issy-les-Moulineaux (FR)
(72) Inventor: DORÉ, Renaud, 35576 CESSON-SÉVIGNÉ (FR); GALPIN, Franck, 35576 CESSON-SÉVIGNÉ (FR); VANDAME, Benoît, 35576 CESSON-SÉVIGNÉ (FR)
(74) Representative: Browaeys, Jean-Philippe

(57) **Abstract**

The present disclosure generally relates to a gaze tracking device. Gaze tracking is a process of measuring either the point of regard or the motion of an eye relatively to the head of a person. A gaze tracking device is a device capable of measuring eye positions and eye movement. In order to track the gaze of a person, infra-red light is projected into the user's eye. Utilizing the primary Purkinje reflection and the pupil-masked reflection, the position of the eye of the user is determined. The gaze tracking devices running such a tracking method offer a limited field of view due to the illumination scheme combined with the geometry of the reflected images. It is proposed a gaze tracking device embedding a light-field camera. Such a gaze tracking device may be embedded in a head mounted device.

## Description

### TECHNICAL FIELD

The present disclosure generally relates to a gaze tracking device capable of providing a reliable and accurate tracking of the gaze of a user, among others for users with narrow eye opening.

### BACKGROUND

Gaze tracking is a process of measuring either the point of regard or the motion of an eye relatively to the head of a person. A gaze tracking device is a device capable of measuring eye positions and eye movement.

As disclosed in patent application WO 2013/167864, gaze tracking is a key feature of Head Mounted Devices or HMD for it can extend the ability of a user of such a HMD to gaze at an object located beyond the head mobility limits. One gaze tracking technology consists in projecting infra-red light into the user's eye and utilizing the primary Purkinje reflection and the pupil-masked reflection in order to determine the position of the eye of the user of the HMD. This method consists in tracking a relative motion of reflected images in order to establish a vector characterizing a point of regard of the user by means of beam splitters located in front of the user's eye. This results in bulky gaze tracking devices difficult to embed in a HMD. Another limitation of this method is the field of view which is limited due to the illumination scheme combined with the geometry of the reflected images.

The present invention has been devised with the foregoing in mind.

### SUMMARY OF INVENTION

A first aspect of the invention concerns a gaze tracking device comprising :
- a plurality of light sources arranged to project infra-red light on a surface of an eyeball and
- a light-field camera for capturing the infra-red light reflected off the surface of the eyeball.

In an embodiment of the gaze tracking device according to the invention, the light sources are located in a periphery of a field of view of the eyeball.

In an embodiment of the gaze tracking device according to the invention, the light-field camera is located in a periphery of a field of view of the eyeball.

In an embodiment of the gaze tracking device according to the invention, the light sources emit a polarized infra-red light.

In an embodiment of the gaze tracking device according to the invention, at least a micro-lens of a micro-lens array of the light-field camera is equipped with a polarizing filter.

A second aspect of the invention concerns a head mounted device comprising at least one gaze tracking device comprising:
- a plurality of light sources arranged to project infra-red light on a surface of an eyeball and
- a light-field camera for capturing the infra-red light reflected off the surface of the eyeball.

According to an embodiment of the head mounted device according to the invention, the light sources are located on a rim of a frame of the head mounted device.

According to an embodiment of the head mounted device according to the invention, the light-field camera is located on the rim of the frame of the head mounted device.

According to an embodiment of the head mounted device according to the invention, the light-field camera is embedded on a side-piece of the frame of the head mounted device.

Some processes implemented by elements of the invention may be computer implemented. Accordingly, such elements may take the form of an entirely hardware embodiment, an entirely software embodiment (including firmware, resident software, micro-code, etc.) or an embodiment combining software and hardware aspects that may all generally be referred to herein as a "circuit", "module" or "system'. Furthermore, such elements may take the form of a computer program product embodied in any tangible medium of expression having computer usable program code embodied in the medium.

Since elements of the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or RF signal.

### BRIEF DESCRIPTION OF THE DRAWINGS

Embodiments of the invention will now be described, by way of example only, and with reference to the following drawings in which:
FIG. 1 represents a gaze tracking device according to an embodiment of the invention,
FIG. 2 represents the micro-lenses of the micro-lens array of the light-field camera of the gaze tracking device according to an embodiment of the invention,
FIG. 3 is a schematic block diagram illustrating an apparatus for processing light-field data acquired by the light-field camera of the gaze tracking device according to an embodiment of the invention,
FIG. 4 represents a head mounted device embedding gaze tracking devices according to an embodiment of the invention.

### DETAILED DESCRIPTION

As will be appreciated by one skilled in the art, aspects of the present principles can be embodied as a system, method or computer readable medium. Accordingly, aspects of the present principles can take the form of an entirely hardware embodiment, an entirely software embodiment, (including firmware, resident software, micro-code, and so forth) or an embodiment combining software and hardware aspects that can all generally be referred to herein as a "circuit", "module", or "system". Furthermore, aspects of the present principles can take the form of a computer readable storage medium. Any combination of one or more computer readable storage medium(a) may be utilized.

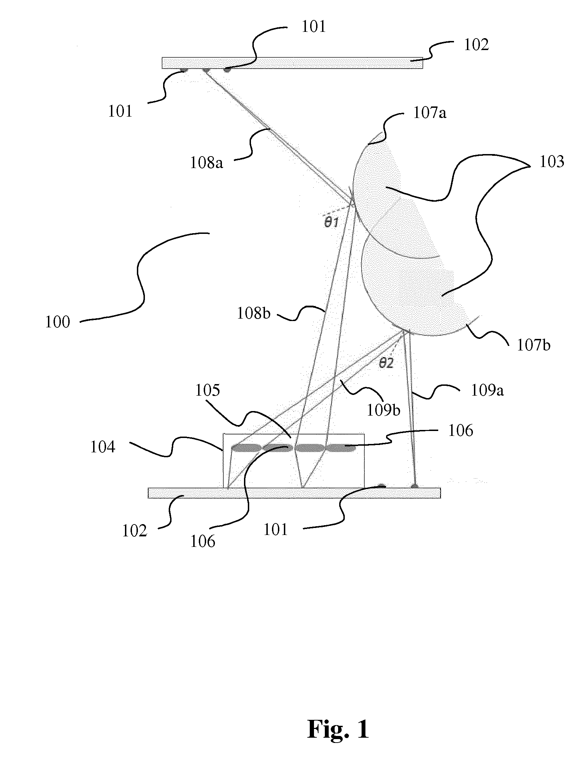

**Figure 1** represents a gaze tracking device 100 according to an embodiment of the invention. Such a gaze tracking device 100 may be mounted on a fixed support comprising for example a chin-rest or may be implemented as a portable device. In the remainder of the description it is assumed that the gaze tracking device 100 is of the portable type, however, the embodiments of the invention described hereinafter may be implemented on a gaze tracking device mounted on a fixed support as well.

The gaze tracking device 100 represented on figure 1 is designed for the left eye of a user. A gaze tracking device adapted for the right eye of a user is symmetrical to the gaze tracking device 100 as shown on figure 1.

The gaze tracking device 100 comprises a plurality of light-sources 101. The light sources 101 are infra-red light sources or IR light sources 101. The IR light sources 101 are located on a frame 102 of the gaze tracking device 100. This way, the IR light sources 101 are not in the field of view of the eye 103 of a user of the gaze tracking device 100 since the IR light sources 101 are located in the periphery of the field of view of the eye 103. In an embodiment of the invention, the IR light sources 101 may be openings such as discs, rectangles, etc.

A light-field camera 104 is embedded in the frame 102 of the gaze tracking device 100. Thus, as for the IR light sources 102, the light-field camera 104 is located in the periphery of the field of view of the eye 103. The light-field camera 104 comprises a micro-lens array 105 comprising a plurality of micro-lenses 106.

The disc portion 107a represents the eye 103 looking to the right. The IR light 108a emitted by an IR light source 101 reflects on the eye looking right 107a with an incidence angle *θ1*. The reflected IR light 108b is captured by the light-field camera 104 through a micro-lens 106.

The disc portion 107b represents the eye 103 looking to the left. The IR light 109a emitted by an IR light source 101 reflects on the eye looking left 107b with an incidence angle *θ2*. The reflected IR light 109b is captured by the light-field camera 104 through a micro-lens 106.

In order to increase a signal-to-noise ratio in the images captured by the light-field camera 104 and thus offering an increase in the accuracy of the gaze tracking information obtained, it is interesting to generate as many reflections of the IR light on the eye as possible. To achieve this goal, IR light sources 101 may be located all around the frame of the gaze tracking device 100 in such a pattern that the IR light emitted by an IR light source 101 is captured by at least one pixel of a sensor of the light-field camera 104.

In another embodiment of the invention, in order to increase the sensibility of the measurements of the gaze of a user, additional information related to a vector normal to the eye 103 surface while IR light is reflecting on the surface of the eye 103 is used. Indeed, the knowledge of vectors normal to the surface of the eye 103 enables to compute an orientation of the eye 103.

Such piece of information related to a vector normal to the surface of the eye 103 is obtained by polarizing the IR light.

In a first embodiment of the invention, the IR light sources 101 emit a polarized IR light. The polarization of the IR light may be achieved by equipping the IR light sources 101 with polarizing filters.

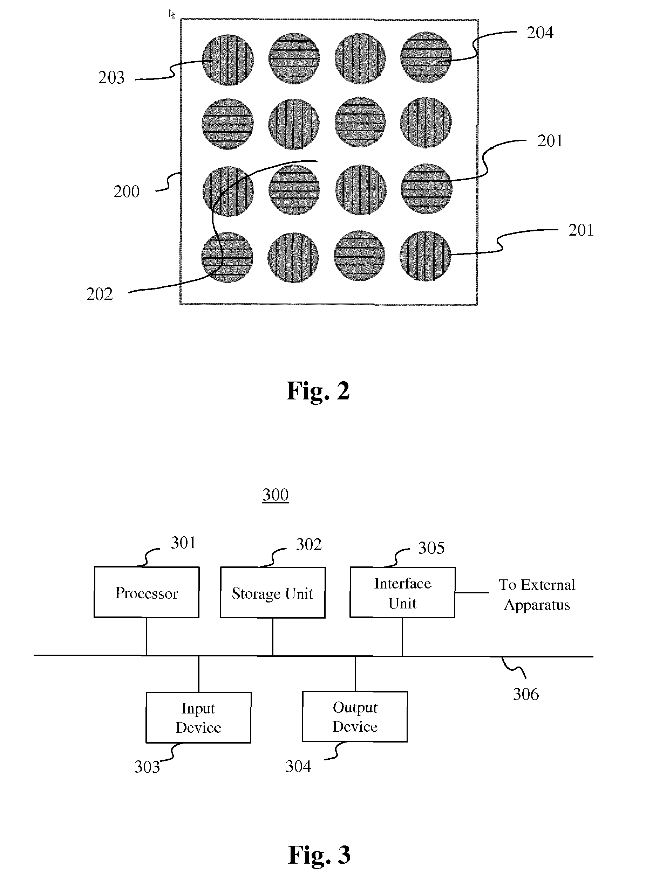

In a second embodiment of the invention represented on **figure 2**, the micro-lenses 201 of the micro-lens array 202 of the light-field camera 200 are equipped with polarizing filters. For example the micro-lenses 201 are equipped with two different types of polarizing filters 203, 204. For example, the polarizing filters 203, 204 may be of the linear polarization type, the polarizations of the polarizing filters 203, 204 being orthogonal to each other. The polarizing filters 203, 204 may also be of the circular polarization type, the polarizations of the polarizing filters 203, 204 being in reverse sense to each other.

The reflection on the surface of the eyeball of a non-polarized IR light emitted by the IR light sources 101 may provide a natural polarization. Indeed when the incidence angle of the emitted IR light is targeted to be equal to the Brewster angle, the polarization of the reflected IR light is close to a parallel polarization, i.e. the polarization of the reflected IR light is orthogonal to the plan defined by the incident IR light and the reflected IR light. The Brewster angle is defined according to the normal vector to the surface of the eyeball on the location where the reflection of the IR light on the eyeball takes place and only depends of the index of the eyeball transparent medium material, considering that the other medium is air. The value of the Brewster angle is not measured per se, only the effects on light are detected through polarization effects.

Thus, in another embodiment of the invention, some of the IR light sources 101 emit a polarized IR light while other IR light sources 101 emit a non-polarized IR light. The IR light sources emitting a non-polarized IR light are selected based on the incidence angle of the IR light emitted and the knowledge that depending on this incidence angle the reflection of the incident IR light on the eyeball results in a natural polarization of the reflected IR light.

In another embodiment of the invention, the selection of the IR light sources 101 emitting a polarized IR light is dynamic and is based on the current position of the eye of the user. Thus, depending on the current position of the eye of the user, a given IR light source 101 emits or does not emit a polarized IR light.

In order to determine the position of the eye of the user, information related to the IR light captured by the light-field camera 104, 200, are transmitted to an image processing device. In an embodiment of the invention, the image processing device and the gaze tracking device 100 are embedded in a same apparatus such as a head mounted device or HMD. In another embodiment of the invention, the image processing device and the gaze tracking device 100 are two distinct devices remote from each other. The information related to the IR light captured by the light-field camera 104 of the gaze tracking device 100 are transmitted to the image processing device via cable or wireless communication. In such an embodiment of the invention, the gaze tracking device 100 is embedded in a head mounted device while the image processing device is for example embedded in a computer.

Figure 3 is a schematic block diagram illustrating an example of an apparatus for processing light-field data acquired by the light-field camera 104 of the gaze tracking device 100 according to an embodiment of the present invention.

The apparatus 300 comprises a processor 301, a storage unit 302, an input device 303, a display device 304, and an interface unit 305 which are connected by a bus 306. Of course, constituent elements of the computer apparatus 300 may be connected by a connection other than a bus connection.

The processor 301 controls operations of the apparatus 300. The storage unit 302 stores at least one program to be executed by the processor 301, and various data, including light-field data acquired by the light-field camera 104 or provided by the gaze tracking device 100, parameters used by computations performed by the processor 301, intermediate data of computations performed by the processor 301, and so on. The processor 301 may be formed by any known and suitable hardware, or software, or a combination of hardware and software. For example, the processor 301 may be formed by dedicated hardware such as a processing circuit, or by a programmable processing unit such as a CPU (Central Processing Unit) that executes a program stored in a memory thereof.

The storage unit 302 may be formed by any suitable storage or means capable of storing the program, data, or the like in a computer-readable manner. Examples of the storage unit 302 include non-transitory computer-readable storage media such as semiconductor memory devices, and magnetic, optical, or magneto-optical recording media loaded into a read and write unit. The program causes the processor 301 to perform a learning process and a classifying process.

The input device 303 may be formed by a keyboard, a pointing device such as a mouse, or the like for use by the user to input commands. The output device 304 may be formed by a display device to display, for example, a Graphical User Interface (GUI). The input device 303 and the output device 304 may be formed integrally by a touchscreen panel, for example.

The interface unit 305 provides an interface between the apparatus 300 and an external apparatus. The interface unit 305 may be communicable with the external apparatus via cable or wireless communication. In an embodiment, the external apparatus may be a head mounted device embedding the gaze tracking device 100 or the gaze tracking device 100 itself. In this case, light-field data acquired by the light-field camera 104 of the gaze tracking device 100 can be input from the gaze tracking device 100 to the apparatus 300 through the interface unit 305, then stored in the storage unit 302.

In this embodiment the apparatus 300 is exemplary discussed as it is separated from the gaze tracking device 100 and they are communicable each other via cable or wireless communication.

The learning process consists in a training period during which a plurality of eye positions are browsed, an example of learning process may rely on the use of a neural network or any other machine learning processes which would be efficient and accurate. Thus during the training period, the data related to the IR light emitted by the IR light sources 101 captured by the light-field camera 104 after the IR light is reflected by the eye are stored in the storage unit 302 of the apparatus 300 for a plurality of eye positions. These stored positions may be determined for example through the use of a moving controlled target or any other calibration means. A pattern is defined as a plurality of reflection light points within the multiple images captured by the light field camera 104, the position in the captured images as well as the intensity of each of the reflection light points are stored in the storage unit 302 of the apparatus 300.

Then the processor 301 runs an identification process determining an estimate position of the eye. The identifying process is executed in real time by the processor 301 after the training period. Using the results of the learning process, i.e. the reflection patterns of the IR lights emitted by the IR light sources 101 stored in the storing unit 302, it is possible to determine the position of the eye of the user in real time.

The gaze tracking device 100 according to the different embodiments of the invention offers information related to the captured IR light which once processed enable the tracking of the gaze in an accurate and reliable way especially for eyes having a narrow opening such as Asian eyes. This is made possible due to the use of a light-field camera 100 which introduces spatial disparity. The accuracy of the gaze tracking is increased by introducing a disparity in polarization in addition to the spatial disparity.

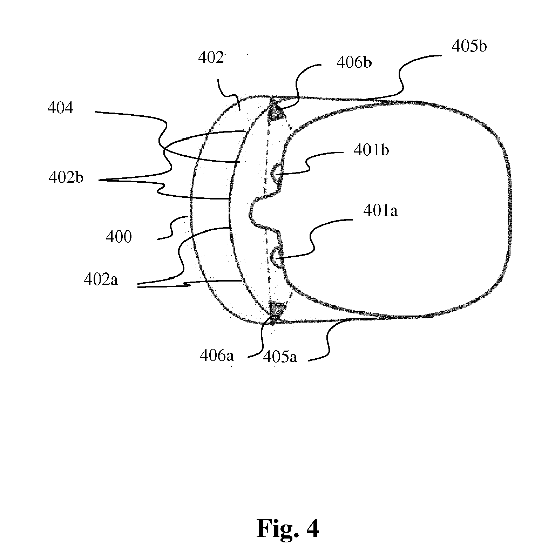

Figure 4 represents a head mounted device 400 embedding two gaze tracking devices for determining the position of the left eye 401 a and the right eye 401 b respectively of a user of the head mounted device 400.

The gaze tracking devices comprise a plurality of light-sources 402a and 402b. The light sources 402a, 402b are IR light sources. The IR light sources 402a, 402b are located on a frame 403 of the head mounted device 400. In an embodiment of the head mounted device 400 according to the invention, the IR light sources 402a, 402b are embedded in the rim 404 of the frame 403 of the head mounted device 400. This way, the IR light sources 402a, 402b are not in the field of view of the eyes 401 a, 401 b of the user of the head mounted device 400. In another embodiment of the invention, the IR light sources 402a, 402b are also embedded in the side-pieces 405a, 405b of the frame 403 of the head mounted device 400.

In an embodiment of the invention, in order to improve the spatial sampling of the gaze tracking device, secondary IR light sources (not represented on the figures) are embedded in the head mounted device 400. The IR light emitted by the secondary IR light sources firstly reflects on a main lens or a main display of the head mounted device 400. In an embodiment of the invention, the secondary IR light sources may be openings presenting an ovoid geometry or may be grids.

Light-field cameras 406a, 406b are embedded in the frame 403 of the head mounted device 400. Thus, as for the IR light sources 402a, 402b, the light-field cameras 406a, 406b are located in the periphery of the field of view of the eyes 401 a, 401 b. The light-field cameras 406a, 406b comprise a micro-lens array comprising a plurality of micro-lenses.

In another embodiment of the head mounted device 400, the light-field cameras 406a, 406b are embedded on the side-pieces 405a, 405b of the frame 403 of the head mounted device 400.

In order to increase the sensibility of the measurements of the gaze of a user, in a first embodiment of the head mounted device 400, the IR light sources 402a, 402b emit a polarized IR light. The polarization of the IR light may be achieved by equipping the IR light sources 402a, 402b with polarizing filters.

In a second embodiment of the head mounted device 400, the micro-lenses of the micro-lens array of the light-field cameras 406a, 406b are equipped with polarizing filters.

The reflection on the surface of the eyeball of a non-polarized IR light emitted by the IR light sources 101 may provide a natural polarization. Thus in a third embodiment of the head mounted device 400, some of the IR light sources 402a, 402b emit a polarized IR light while other IR light sources 402a, 402b emit a non-polarized IR light. The IR light sources emitting a non-polarized IR light are selected based on the incidence angle of the IR light emitted and the knowledge that depending on this incidence angle the reflection of the incident IR light on the eyeball results in a natural polarization of the reflected IR light.

In another embodiment of the head mounted device 400, the selection of the IR light sources 402a, 402b emitting a polarized IR light is dynamic and is based on the current position of the eye of the user. Thus, depending on the current position of the eye of the user, a given IR light source 402a, 402b emits or does not emit a polarized IR light.

In order to determine the position of the eye of the user, information related to the IR light captured by the light-field cameras 406a, 406b, are transmitted to an image processing device. In an embodiment of the invention, the image processing device is embedded in the head mounted device 400. In another embodiment of the invention, the image processing device and the head mounted device 400 are two distinct devices remote from each other. The information related to the IR light captured by the light-field cameras 406a, 406b are transmitted to the image processing device via cable or wireless communication.

Although the present invention has been described hereinabove with reference to specific embodiments, the present invention is not limited to the specific embodiments, and modifications will be apparent to a skilled person in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular the different features from different embodiments may be interchanged, where appropriate.

## Claims

1. A gaze tracking device comprising:
- a plurality of light sources arranged to project infra-red light on a surface of an eyeball and
- a light-field camera for capturing the infra-red light reflected off the surface of the eyeball.

2. The gaze tracking device according to claim 1, wherein the light sources are located in a periphery of a field of view of the eyeball.

3. The gaze tracking device according to claim 1 or 2, wherein the light-field camera is located in a periphery of a field of view of the eyeball.

4. The gaze tracking device according to any one of claims 1 to 3, wherein the light sources emit a polarized infra-red light.

5. The gaze tracking device according to any one of claims 1 to 3, wherein at least a micro-lens of a micro-lens array of the light-field camera is equipped with a polarizing filter.

6. A head mounted device comprising at least one gaze tracking device comprising:
- a plurality of light sources arranged to project infra-red light on a surface of an eyeball and
- a light-field camera for capturing the infra-red light reflected off the surface of the eyeball.

7. The head mounted device according to claim 6, wherein the light sources are located on a rim of a frame of the head mounted device.

8. The head mounted device according to claim 6 or 7, wherein the light-field camera is located on the rim of the frame of the head mounted device.

9. The head mounted device according to claim 8, wherein the light-field camera is embedded on a side-piece of the frame of the head mounted device.
